# EUROPEAN PATENT APPLICATION

(11) **EP 1 905 399 A1**
(43) Date of publication of application: **02.04.2008**
(21) Application number: 07116386.9
(22) Date of filing: 14.09.2007
(51) Int. Cl.: A61F 5/00

(54) **Thumb Immobilizer**

(30) Priority: 29.09.2006 US 848031 P
(71) Applicant: Becton, Dickinson & Company, Franklin Lakes, NJ 07417-1880 (US)
(72) Inventor: Ortiz, David, Las Piedras, Puerto Rico 00771 (UM)
(74) Representative: Weber, Thomas

(57) **Abstract**

An orthopedic device for immobilizing a thumb includes a main body having a wrist end and a thumb end. A wrist strap is located on the wrist end of the main body for fastening the wrist end of the main body to the wrist of a user, and a thumb strap is located on the thumb end of the main body for fastening the thumb end of the main body to the wrist of a user. The thumb strap is adapted to encircle a joint of a user's thumb. When the wrist and thumb straps are fastened, the main body substantially immobilizes the thumb.

## Description

### BACKGROUND OF THE INVENTION

### Cross Reference to Related Applications:

This application claims the benefit under 35 U.S.C. § 119(e) of provisional patent application Serial No. 60/848,031, filed September 29, 2007, which is hereby incorporated by reference in its entirety.

### Field of the Invention:

The present invention relates to orthopedic support devices. More particularly, the present invention relates to a molded thumb brace which minimizes movement of the thumb.

### Description Of The Related Art:

Various orthopedic support devices are known for supporting a thumb after an injury. Typically, these devices utilize a splint which attempts to immobilize the thumb so that the thumb is placed into a fixed position. These devices, however, have various drawbacks. For example, one type of thumb support device utilizes a soft, fabric material with metal stays sewn into place to provide rigidity. The metal stays, however, are very rigid, and may not conform to a user's hand. Thus, the rigid metal stays may be uncomfortable for a user. Moreover, conventional devices typically employ two rigid metal stays placed on opposite sides of the thumb, and the stays do not completely enclose the thumb. Therefore, the metal stays still allow movement of the thumb, and the thumb is not totally immobilized.

Another issue with fabric type support devices is that the fabric (or possibly neoprene) material deteriorates relatively quickly. Therefore, the user may need to frequently replace the device.

Accordingly, there is a need for an improved orthopedic support device for supporting a thumb.

### SUMMARY OF THE INVENTION

An object of the present invention is to address at least the above problems and/or disadvantages and to provide at least the advantages described below. Accordingly, an object of the present invention is to provide a thumb immobilizer formed of a semi-rigid laminate material to immobilize a thumb.

Another object of the present invention is to provide a thumb immobilizer which provides sufficient support to a user's thumb to substantially prevent movement of the thumb.

Another object of the present invention is to provide a thumb immobilizer which is convenient for a user to use.

Yet another object of the present invention is to provide a thumb immobilizer which is easy to manufacture.

Still another object of the present invention is to provide a thumb immobilizer formed of a durable material.

In accordance with an aspect of the present invention, an orthopedic device for immobilizing a thumb includes a main body having a wrist end and a thumb end, the wrist end being wider than the thumb end, a wrist strap located on the wrist end of the main body for fastening the wrist end of the main body to the wrist of a user, and a thumb strap located on the thumb end of the main body for fastening the thumb end of the main body to the wrist of a user. When the wrist and thumb straps are fastened, the main body substantially immobilizes the thumb.

In accordance with another aspect of the present invention, an orthopedic device for immobilizing a thumb comprises a main body having a wrist end and a thumb end, a wrist strap located on the wrist end of the main body for fastening the wrist end of the main body to the wrist of a user, and a thumb strap located on the thumb end of the main body for fastening the thumb end of the main body to the thumb of a user. The thumb strap is adapted to encircle a joint of a user's thumb. When the wrist and thumb straps are fastened, the main body substantially immobilizes the thumb.

In accordance with still another aspect of the present invention, a method of forming an orthopedic device for immobilizing a thumb comprises the steps of: (a) forming a main body blank; (b) heating the main body blank to a temperature sufficient to allow the main body blank to be molded; and (c) molding the main body blank into a desired shape. The main body blank comprises a thumb portion disposed at a thumb end of the main body blank, the thumb portion having a first width, a wrist portion disposed at a wrist end of the main body, the wrist portion having a second width, and a tapered portion disposed between the thumb portion and the wrist portion for connecting the thumb portion to the wrist portion.

### BRIEF DESCRIPTION OF THE DRAWINGS

The above and other objects, features, and advantages of certain exemplary embodiments of the present invention will be more apparent from the following description taken in conjunction with the accompanying drawings, in which:

Figure 1 is a perspective view of an exemplary embodiment of a thumb immobilizer, with the straps secured;

Figure 2 is a top plan view of the thumb immobilizer of Figure 1;

Figure 3 is an outside side view of the thumb immobilizer of Figure 1;

Figure 4 is an inside side view of the thumb immobilizer of Figure 1;

Figure 5 is a view of the wrist end of the thumb immobilizer of Figure 1;

Figure 6 is a view of the thumb end of the thumb immobilizer of Figure 1;

Figure 7 is a bottom plan view of the thumb immobilizer of Figure 1;

Figure 8 is a perspective view of the thumb immobilizer of Figure 1, placed on a hand;

Figure 9 is a perspective view of the thumb immobilizer of Figure 1, with the straps open;

Figure 10 is a plan view of a blank used for forming the thumb immobilizer of Figure 1;

Figure 11 is a plan view of the main body of the thumb immobilizer of Figure 1, in a flattened state;

Figure 12 is a plan view of the main body of the thumb immobilizer of Figure 1 with an attached thumb strap, in a flattened state;

Figure 13 is a side view of the thumb immobilizer of Figure 1; and

Figure 14 is a sectional view of an exemplary embodiment of the laminate material used to form the thumb immobilizer of Figure 1.

Throughout the drawings, the same reference numerals will be understood to refer to the same elements, features, and structures.

### DETAILED DESCRIPTION OF EXEMPLARY EMBODIMENTS

The matters defined in the description such as a detailed construction and elements are provided to assist in a comprehensive understanding of the embodiments of the invention and are merely exemplary. Accordingly, those of ordinary skill in the art will recognize that various changes and modifications of the embodiments described herein can be made without departing from the scope and spirit of the invention. Also, descriptions of well-known functions and constructions are omitted for clarity and conciseness.

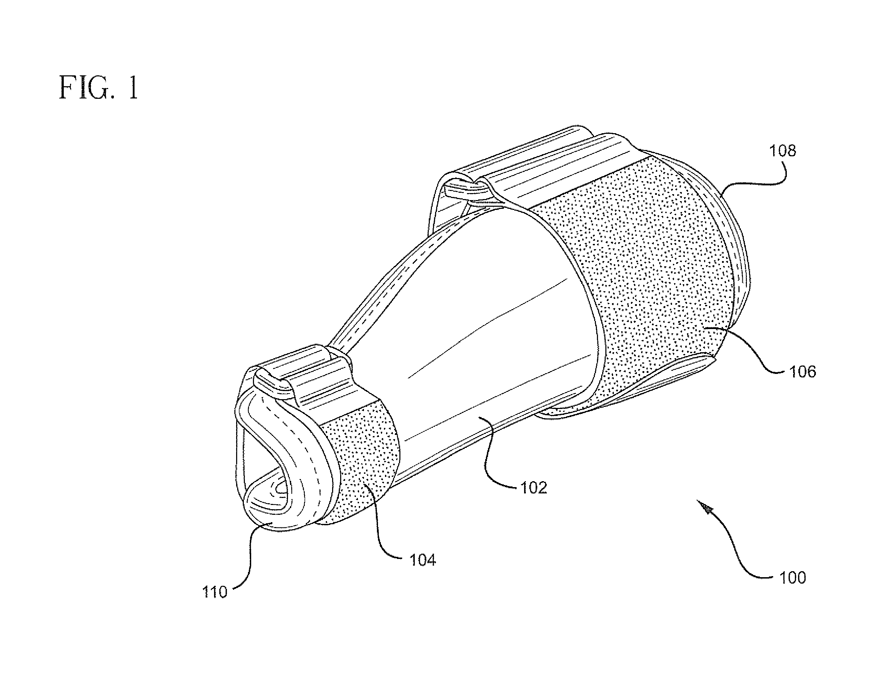

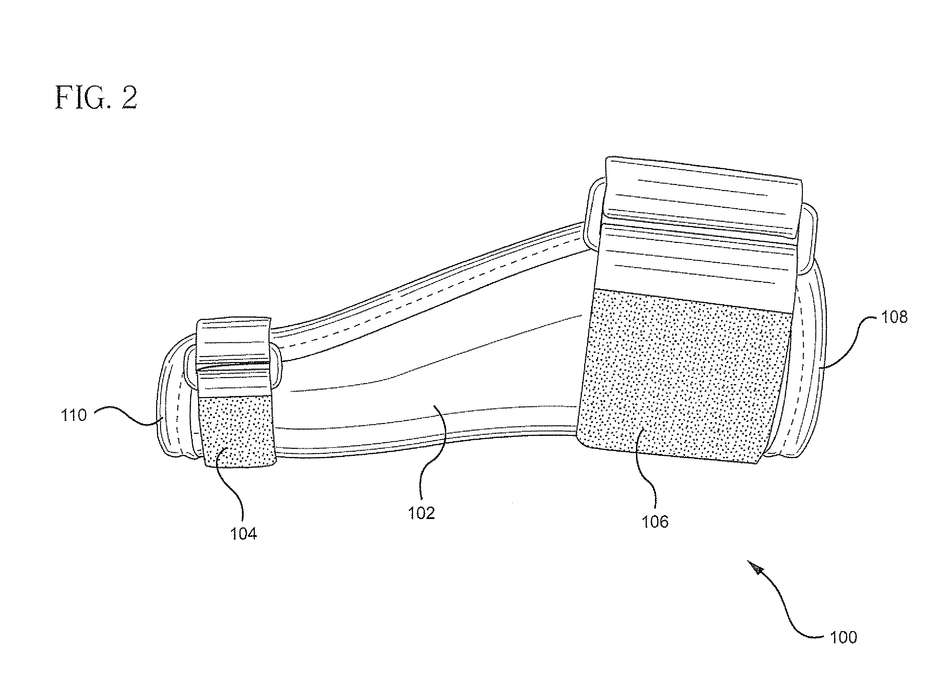

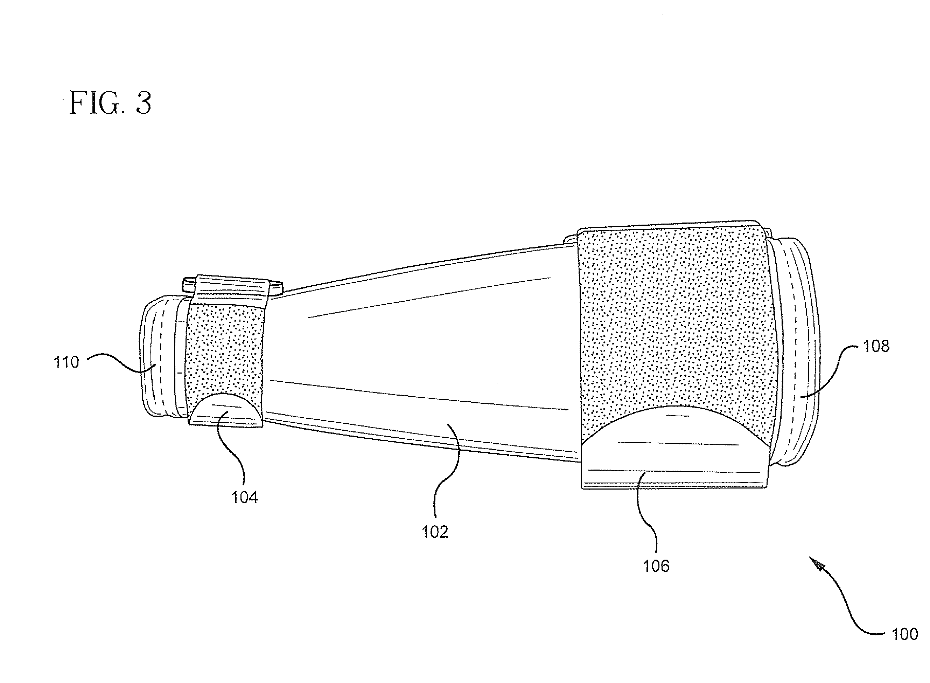

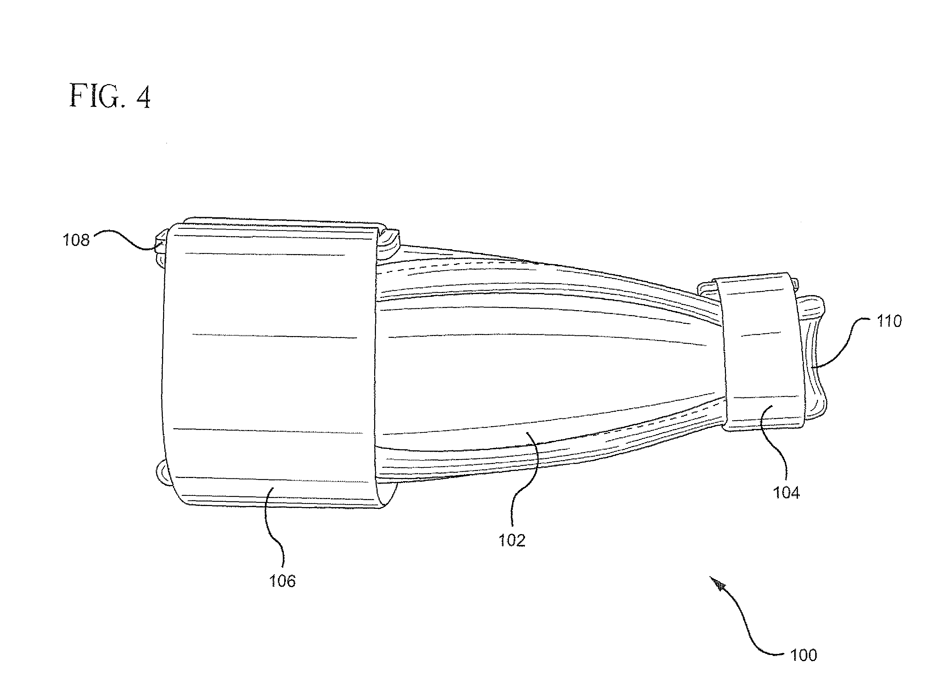

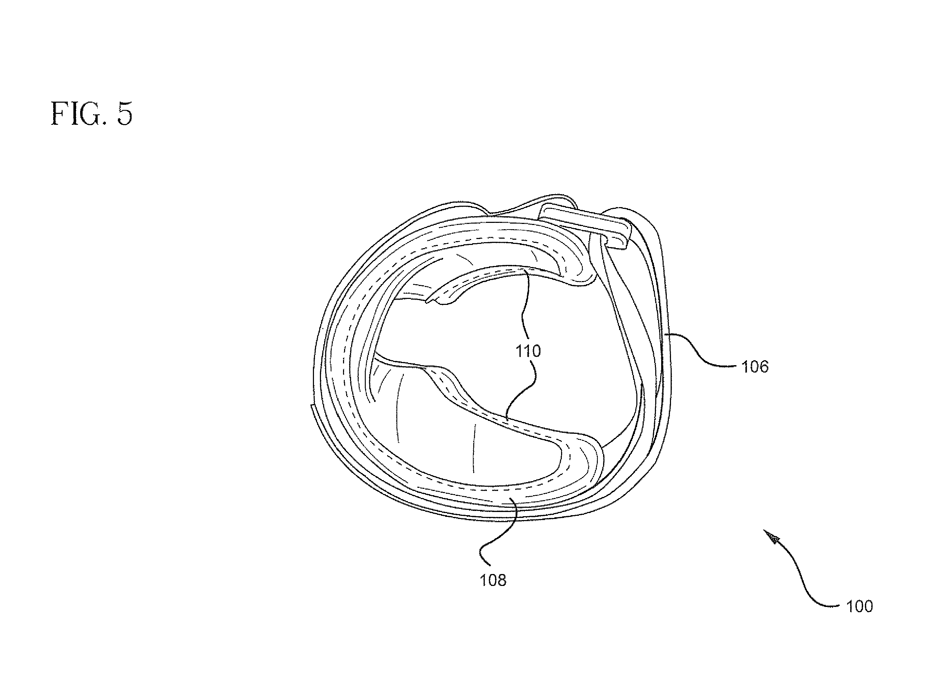

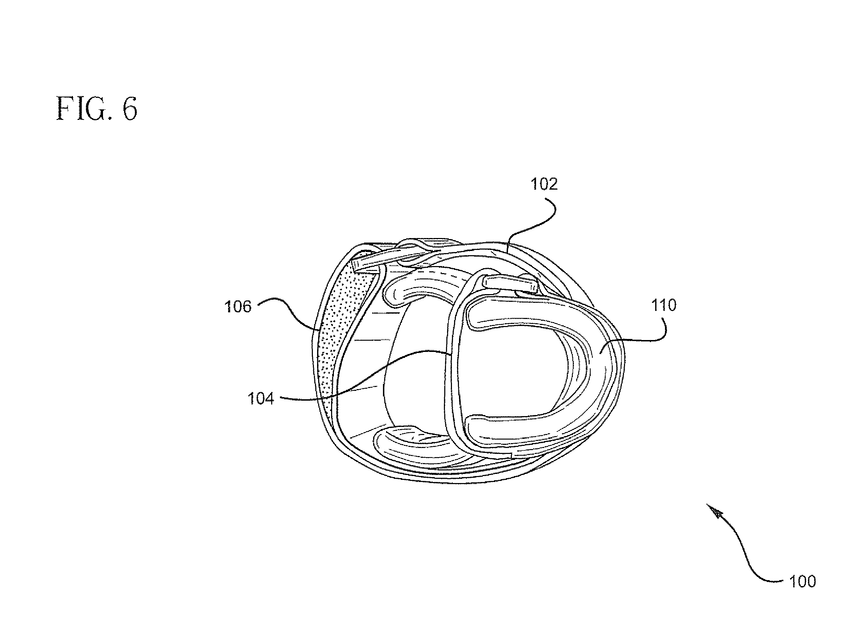

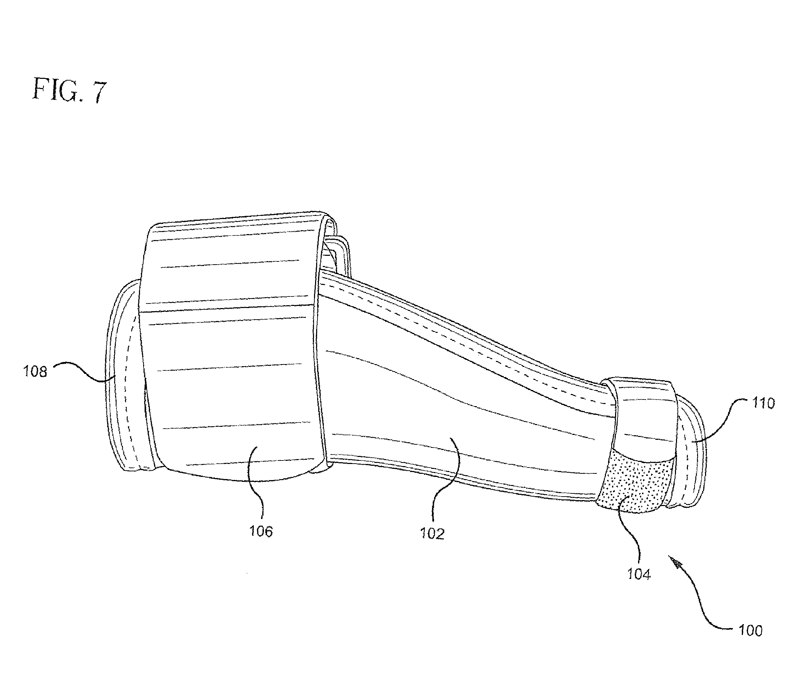

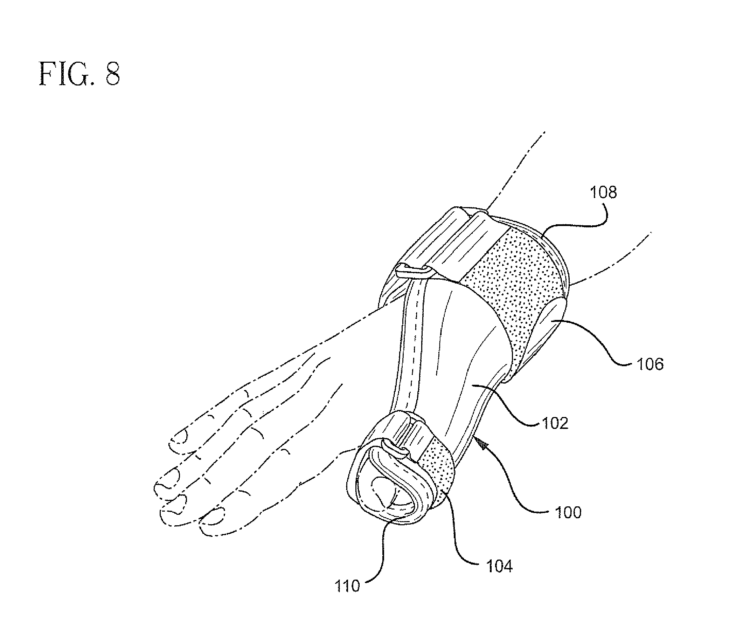

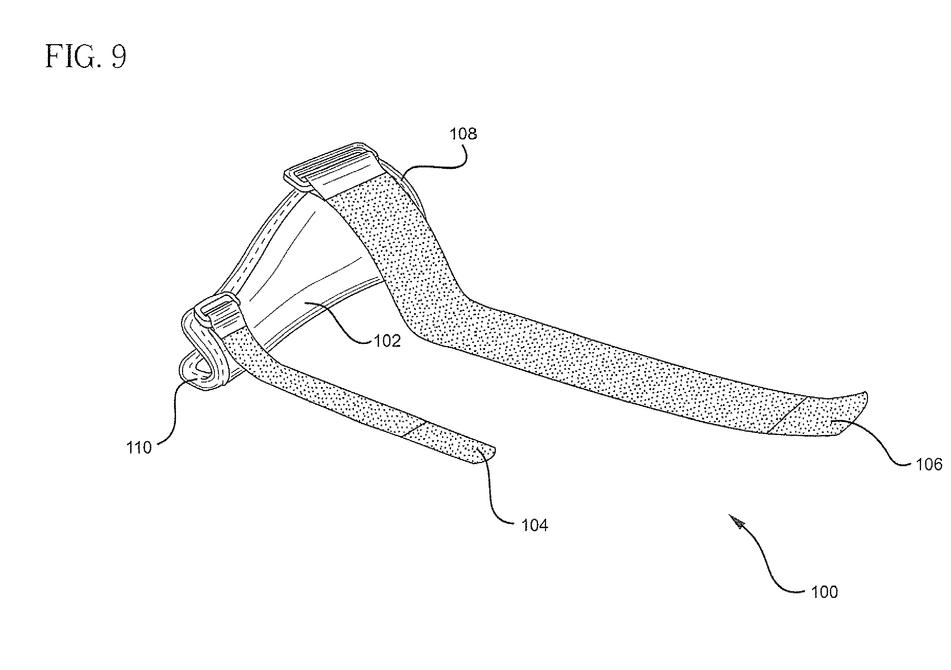

As seen in Figures 1-9, a thumb immobilizer 100 according to an exemplary embodiment of the present invention has a main body 102, a thumb strap 104, and a wrist strap 106. The main body 102 has a wrist end 108 and a thumb end 110, which are configured to embrace a user's wrist and thumb, respectively. The thumb strap 104 and the wrist strap 106 tighten and fasten the main body 102 to the user's wrist and thumb. The main body 102 is formed of a semi-rigid laminate material 118 so that the thumb is substantially immobilized.

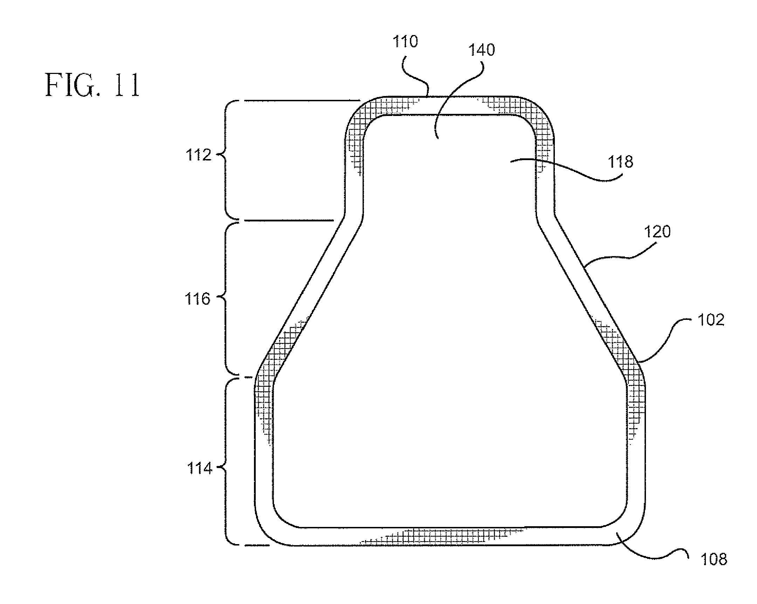

Figure 11 is a plan view of the main body 102 of the thumb immobilizer 100 of Figure 1, in a flattened state prior to molding. As seen in Figure 11, the main body 102 has a thumb portion 112, a wrist portion 114, and a tapered portion 116. The thumb portion 112 is disposed at the thumb end 110 of the main body 102 and, after molding, forms a recess for accommodating a user's thumb. The thumb portion 112 has a first width. The wrist portion 114 is disposed at the wrist end 108 of the main body 102, and, after molding, forms a recess for accommodating a user's thumb. The wrist portion 114 is wider than the thumb portion 112. A tapered portion 116 is disposed between the thumb portion 112 and the wrist portion 114 for connecting the thumb portion 112 to the wrist portion 114.

Preferably, the size of the main body 102 is chosen so that it fits the majority of intended users-that is, it is a "one size fits all" type device. The main body 102 may, however, be provided in different sizes, such as small, medium, and large. In an exemplary embodiment, the thumb portion 112 is approximately 3 1/8" (79.7 mm) wide, and the thumb portion 112 is approximately 6" (152.4 mm) wide. The length of the thumb portion 112 is approximately 1 3/4" (44.5 mm), the length of the wrist portion 114 is approximately 2 1/2" (6.4 mm), and the overall length is approximately 7" (17.8 mm). The thumb immobilizer 100 is not hand specific-that is, the same immobilizer may be used on either the right or left hand.

By using a one-size fits all, non-hand specific device, a single thumb immobilizer may be used for all users. This is more convenient for users because they do not need to choose the proper size of device. Furthermore, it minimizes the possibility of injury due to a user attempting to utilize the wrong size device, such as by forcing a too small device onto a large user's hand, or vice versa.

The wrist portion 114 of the main body 102 is preferably wider than the thumb portion 112 so that the thumb immobilizer 100 provides sufficient support to a user's wrist and thumb to substantially prevent movement of the thumb. Without sufficient support, a user's thumb may move too much, which may lead to discomfort or additional injury. The use of a wide wrist portion 114 also provides sufficient support without requiring excessive tightening of the wrist strap 106, which may be required with a narrower wrist support.

Moreover, by providing a wider wrist portion that contacts the wrist and hand, the thumb immobilizer provides increased comfort to a user and additional stability to the thumb.

The main body 102 is preferably formed of a heat formable laminate material 118, which will be described in detail below. A binding 120 is located around the edge of the main body 102 to conceal the edges of the laminate material 118 and to make the main body 102 more aesthetically pleasing. The binding 120 also prevents the edges of the laminate material 118 from scraping against a user's skin. The binding 120 may be a fabric (for example, polyester) material which is stitched into place.

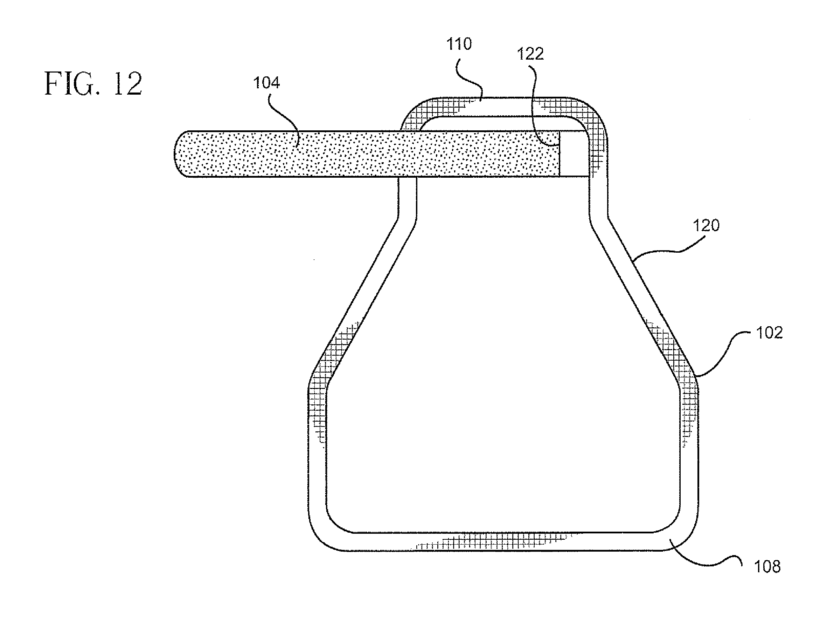

As seen in Figure 12, the thumb strap 104 is fastened to the thumb portion 112 of the main body 102. Preferably, the thumb strap 104 is fastened to the main body 102 by stitching the strap to the main body 102 across a stitching line 122. The thumb strap 104 is preferably oriented substantially orthogonally to the longitudinal direction of the main body 102. When positioned in this manner, the thumb strap 104 basically encircles a user's thumb, thereby immobilizing the user's thumb. Even more preferably, the thumb strap 104 is configured to encircle the top joint of a user's thumb to provide additional support for the thumb.

The thumb strap 104 may be made of a hook and loop material to allow a user to easily adjust the thumb strap 104. A buckle 126 may be provided at one end of the thumb strap 104 so that the thumb strap 104 may be passed through the buckle 126 and looped back onto itself. The buckle 126 may be attached to the thumb strap 104 by forming a loop in the thumb strap 104 that encloses the buckle 126, and stitching the loop closed. The thumb strap 104 may be approximately 3/4" (19.0 mm) wide.

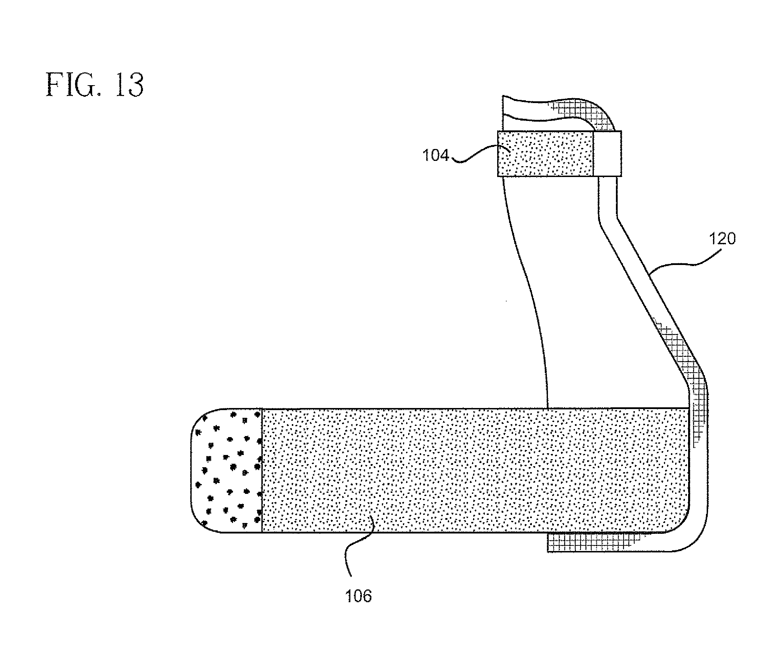

As seen in Figure 13, the wrist strap 106 is fastened to the wrist portion 114 of the main body 102. Like the thumb strap 104, the wrist strap 106 may be fastened to the main body 102 by stitching the strap to the main body 102 across a stitching line (not illustrated). The thumb strap 104 is preferably oriented substantially orthogonally to the longitudinal direction of the main body 102 so that it may be used to encircle a user's wrist.

The wrist strap 106 may be made of a hook and loop material to allow a user to easily adjust the strap. A buckle 128 may be provided at one end of the wrist strap 106 so that the wrist strap 106 may be passed through the buckle 128 and looped back onto itself. The buckle 128 may be attached to the wrist strap 106 by forming a loop in the wrist strap 106 that encloses the buckle 128, and stitching the loop closed. The wrist strap 106 may be approximately 2" (50.8 mm) wide.

Figure 13 is a side view of the assembled and molded thumb immobilizer 100. In Figure 13, the thumb immobilizer 100 is open to the right. That is, a user would insert his or her hand from the right side. The left side of the thumb immobilizer 100 (as seen in Figure 13) is curved so that it is generally shaped to accommodate the curve between a user's wrist and thumb. Therefore, the thumb immobilizer 100 fits snugly against a user's wrist and thumb. The right side of the thumb immobilizer 100 (as seen in Figure 13) is generally shaped so that it minimizes interference with a user's hand, while still maintaining the required stiffness and rigidity to immobilize a user's thumb.

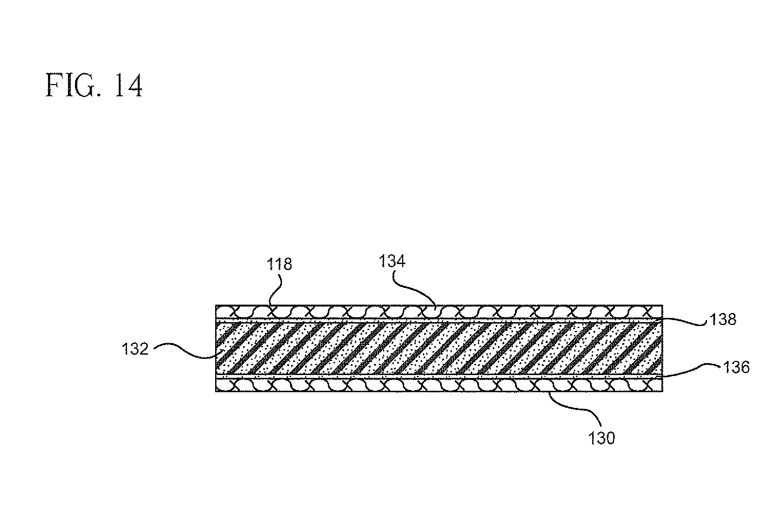

Figure 14 is a schematic cross-section of an example of a laminate material 118 used to form the main body 102 of the thumb immobilizer 100. The laminate material is semi-rigid and relatively durable. Because the laminate material is relatively durable, it is long-lasting, and will not degrade excessively during use.

The laminate material 118 is manufactured in a sheet form, and comprises three layers, an interior layer 130, an internal layer 132, and an exterior layer 134. The interior layer 130 forms the interior surface of the thumb immobilizer 100, and is placed adjacent to a user's hand. The interior layer 130 may be formed of a first fabric material, such as a micro-polyester. Preferably, the interior layer 130 is treated with an anti-microbial agent to minimize odors and bacterial growth. One suitable anti-microbial agent is available under the trademark SANITIZED® from Sanitized AG, Burgdorf, Switzerland.

The interior layer 130 of the laminate material 118 is bonded to the internal layer 132 of the laminate material 118 by a first adhesive layer 136. The first adhesive layer 136 may be formed of a non-clickable polyester polyurethane foam.

The internal layer 132 of the laminate material 118 is preferably formed of a cross-linked polyethylene foam. The polyethylene foam may be a chemically or an irradiation cross-linked polyethylene foam. The internal layer 132 may be approximately 0.125" (3.2 mm) thick.

The exterior layer 134 of the laminate material 118 comprises a second fabric material, such as a polyester material. The exterior layer 134 of the laminate material 118 is bonded to the internal layer 132 of the laminate material 118 by a second adhesive layer 138. The second adhesive layer 138 may be formed of a non-clickable polyester polyurethane foam.

The overall finished thickness of the laminate material 118 is approximately 0.170" (4.3 mm). At room temperature, the laminate material 118 is semi-rigid, so that it provides support to the thumb immobilizer 100. The laminate material 118 is heat formable at relatively low temperatures (i.e. below 200° F (93°C)). That is, it may be formed by heating the material, and then molding the material.

One suitable laminate material 118 for manufacturing a thumb immobilizer 100 is Model No. 5100727 available from DELA Incorporated of Ward Hill, Massachusetts. Appendix A details the components of the laminate material, as well as the details of the other materials used in the construction of the thumb immobilizer.

The following two tables detail the material construction of an exemplary embodiment of the 0.170" polyethylene foam laminate material used in the manufacture of the thumb immobilizer. The specifications of the components as well as the finished laminate are described.

| **COMPONENT** | | **REQUIREMENT** |
|---|---|---|
| *Component 1* | | |
| **Micro Polyester (Safer Sport 98026)** | | |
| | Content: | 100% Micro Polyester |
| | Finish: | |
| | Antimicrobial: Sanitized T 96-21 | 2% w/w |
| | Construction: | Pique |
| | Weight: | 5.4 ounces/yard² ± 5% |
| | Color: | Black |
| *Component 2* | | |
| **Adhesive Layer to Bond Components 1 and 3** | | |
| | Content: | Non-clickable Polyester Polyurethane Foam |
| | Color: | Charcoal |
| *Component 3* | | |
| **Crosslinked Polyethylene Foam (XPE)** | | |
| | Content: | Crosslinked Polyethylene Foam |
| | Thickness: | 0.125 " ± 10% |
| | Working Temperature: | -65°F - 210°F |
| | Color | Flesh tone or White |
| *Component 4* | | |
| **Same as Component 2** | | |
| *Component* 5 | | |
| **Interlock** | | |
| | Yarn: | 100% Polyester |
| | Construction: | Circular Knit |
| | Color: | Grey #15534 |
| | Weight: | 3.15 oz. / yard² ± 5% |
| **Finished Laminate of Components 1-5** | | |
| | Thickness (overall): | 0.170" - 0.200" |
| | Width: | 58" ± 1 " |

**TAPE 7/8IN W HERRING BONE BLACK**

| PROPERTY | SPECIFICATION AND TOLERANCE |
|---|---|
| WIDTH | 7/8IN ± 1/16IN |
| STRETCH | N/A |
| PICKS PER INCH | 30 ± 2 |
| COLOR | BLACK |
| MATERIAL PACKAGE | FESTOONED |
| MATERIAL CONDITION | FREE OF RAVEL AND/OR FRAY |

| MATERIAL COMPOSITION | |
|---|---|
| COMPONENT DESCRIPTION | ENDS |
| 2/150 POLYESTER BLACK (WARP YARN) | 13 |
| 2/32 COTTON BLACK (FILL YARN) | 6 |

The following table details the material construction of an exemplary embodiment of the hook and loop strap material used in the thumb closure in the thumb immobilizer. The loop portion of the strap has a plastic buckle welded to the end to facilitate the closing of the hook material over the loop material.

| **COMPONENT** | **REQUIREMENT** |
|---|---|
| Material Type | Nylon |
| Hooks per Inch | 187 |
| Average Shear Strength | 800 g/cm² |
| Average Peel Strength | 150 g/cm |
| Width | ¾" ± 0.08" (19.05 mm ± 4 mm) |
| Length | 7" ± 0.5" (177.8 mm ± 13 mm) |
| Plastic Buckle | ¾" D-type buckle welded to the strap |
| Strap Color | Black |

The following table details the material construction of an exemplary embodiment of the hook and loop strap material used in the wrist closure in the thumb immobilizer. The loop portion of the strap has a plastic buckle welded to the end to facilitate the closing of the hook material over the loop material.

| **COMPONENT** | **REQUIREMENT** |
|---|---|
| Material Type | Nylon/Spandex |
| Hooks per Inch | 544 |
| Average Shear Strength | 369 g/cm² |
| Average Peel Strength | 135 g/cm |
| Tension of Loop (stretch) | 180% |
| Width | 2" ± 0.08" (50.8 mm ± 4 mm) |
| Length | 15" ± 0.5" (381 mm ± 13 mm) |
| Plastic Buckle | 2" D-type buckle welded to the strap |
| Color | Black |

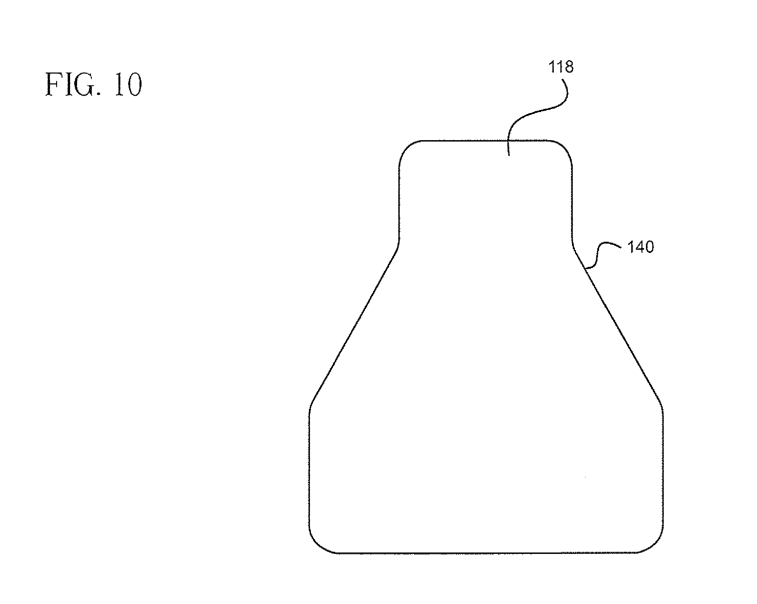

A method of manufacturing a thumb immobilizer 100 in accordance with an exemplary embodiment of the present invention will now be described with reference to Figures 10-13. Initially, a blank 140 of laminate material 118 is formed in the configuration shown in Figure 10. Next, the binding 120 material is applied to the edges of the blank 140. Preferably, the binding 120 material is applied by folding a fabric tape in half, and inserting the edges of the blank 140 into the folded tape. The tape is then stitched to the laminate material 118 to fasten the tape to the blank 140, and form the binding 120. The thumb strap 104 is then attached to the blank 140 by, for example, stitching across a stitching line 122, as shown in Figure 12. Similarly, the wrist strap 106 is attached to the blank 140 by, for example, stitching across a stitching line (not illustrated).

After the straps 104, 106 have been attached to the blank 140, the assembly is heated to a suitable working temperature so that it becomes flexible. As stated before, the temperature is preferably less than 200° F (93°C). Once heated, the assembly is placed over a mold that is shaped in the desired form. The laminated material takes on the shape of the mold. The assembly is allowed to cool to room temperature. Once the assembly is at room temperature, the blank 140 of laminate material 118 retains the molded shape. Accordingly, the blank 140 has been formed into a thumb immobilizer 100, and the thumb immobilizer 100 is ready for packaging, sale, and use.

The use of the thumb immobilizer 100 will now be described. Initially, the straps 104, 106 of the thumb immobilizer 100 are released so that the thumb immobilizer 100 is open. A user then places the thumb immobilizer 100 on his or her wrist and thumb in the desired location. The user may then tighten the thumb strap 104 to fasten the thumb portion 112 of the thumb immobilizer 100 to the user's thumb. Preferably, the thumb strap 104 encircles the user's thumb, particularly the top joint of the thumb, to add additional support for the thumb. Next, the user tightens the wrist strap 106 to fasten the wrist portion 114 of the thumb immobilizer 100 to the user's wrist. The thumb immobilizer 100 now minimizes movement of the thumb. The user may, of course, fasten the wrist strap 106 first, and then fasten the thumb strap 104.

While the invention has been shown and described with reference to certain embodiments thereof, it will be understood by those skilled in the art that various changes in form and details may be made therein without departing from the spirit and scope of the invention as defined by the appended claims and their equivalents.

## Claims

1. An orthopedic device for immobilizing a thumb, comprising:
a main body having a wrist end and a thumb end, the wrist end being wider than the thumb end;
a wrist strap located on the wrist end of the main body for fastening the wrist end of the main body to the wrist of a user; and
a thumb strap located on the thumb end of the main body for fastening the thumb end of the main body to the wrist of a user,
wherein when the wrist and thumb straps are fastened, the main body substantially immobilizes the thumb.

2. An orthopedic device according to claim 1, wherein the main body comprises a laminate material.

3. An orthopedic device according to claim 2, wherein the laminate material comprises:
an interior layer for placement adjacent to a user's hand, the interior layer comprising a first fabric material;
an internal layer bonded to the interior layer, the internal layer comprising polyethylene foam;
an exterior layer bonded to the exterior of the interior layer, the exterior layer comprising a second fabric material.

4. An orthopedic device according to claim 3, wherein the interior layer is a micro-polyester.

5. An orthopedic device according to claim 3, wherein the interior layer is treated with an anti-microbial agent.

6. An orthopedic device according to claim 2, wherein the laminate material is a heat formable material.

7. An orthopedic device according to claim 1, wherein the thumb strap is configured to encircle a user's thumb.

8. An orthopedic device according to claim 1, wherein the thumb strap is configured to encircle the first joint in a user's thumb.

9. An orthopedic device according to claim 1, wherein the main body comprises:
a thumb portion disposed at the thumb end of the main body, the thumb portion having a first width;
a wrist portion disposed at the wrist end of the main body, the wrist portion having a second width; and
a tapered portion disposed between the thumb portion and the wrist portion for connecting the thumb portion to the wrist portion.

10. An orthopedic device for immobilizing a thumb, comprising:
a main body having a wrist end and a thumb end;
a wrist strap located on the wrist end of the main body for fastening the wrist end of the main body to the wrist of a user; and
a thumb strap located on the thumb end of the main body for fastening the thumb end of the main body to the thumb of a user, the thumb strap being adapted to encircle a joint of a user's thumb,
wherein when the wrist and thumb straps are fastened, the main body substantially immobilizes the thumb.

11. An orthopedic device according to claim 10, wherein the main body comprises a laminate material.

12. An orthopedic device according to claim 11, wherein the laminate material comprises:
an interior layer for placement adjacent to a user's hand, the interior layer comprising a first fabric material;
an internal layer bonded to the interior layer, the internal layer comprising polyethylene foam;
an exterior layer bonded to the exterior of the interior layer, the exterior layer comprising a second fabric material.

13. An orthopedic device according to claim 12, wherein the interior layer is a micro-polyester.

14. An orthopedic device according to claim 12, wherein the interior layer is treated with an anti-microbial agent.

15. An orthopedic device according to claim 11, wherein the laminate material is a heat formable material.

16. An orthopedic device according to claim 10, wherein the wrist end of the main body is wider than the thumb end of the main body.

17. An orthopedic device according to claim 10, wherein the main body comprises:
a thumb portion disposed at the thumb end of the main body, the thumb portion having a first width;
a wrist portion disposed at the wrist end of the main body, the wrist portion having a second width; and
a tapered portion disposed between the thumb portion and the wrist portion for connecting the thumb portion to the wrist portion.

18. A method of forming an orthopedic device for immobilizing a thumb, the method comprising the steps of:
(a) forming a main body blank, the main body blank comprising:
a thumb portion disposed at a thumb end of the main body blank, the thumb portion having a first width;
a wrist portion disposed at a wrist end of the main body, the wrist portion having a second width; and
a tapered portion disposed between the thumb portion and the wrist portion for connecting the thumb portion to the wrist portion;
(b) heating the main body blank to a temperature sufficient to allow the main body blank to be molded; and
(c) molding the main body blank into a desired shape.

19. A method of forming an orthopedic blank according to claim 18, further comprising the step of:
assembling a thumb strap to the thumb end of the main body blank.

20. A method of forming an orthopedic blank according to claim 18, further comprising the step of:
assembling a wrist strap to the wrist end of the main body blank.
